Europäisches Patentamt

European Patent Office

Office européen des brevets

Numéro de publication: **0 367 647**

**A1**

# DEMANDE DE BREVET EUROPEEN

Numéro de dépôt: **89402814.1**

Int. Cl.5 **G01J 3/50 , A61C 13/08**

Date de dépôt: **12.10.89**

Priorité: **17.10.88 FR 8813635**

Date de publication de la demande:
**09.05.90 Bulletin 90/19**

Etats contractants désignés:
**AT BE CH DE ES FR GB GR IT LI LU NL SE**

Demandeur: **BERTIN & CIE**
**B.P. nr. 3**
**F-78373 Plaisir Cédex(FR)**

Inventeur: **Dordet, Yves**
**Les Althéas Rue Lou Coustéloun**
**F-84120 Pertuis(FR)**
Inventeur: **Decaudin, Jean-Michel**
**24 Allée J.S. Bach**
**F-13880 Velaux(FR)**
Inventeur: **Duret, François**
**Rue Paul Claudel**
**F-38690 Le Grand Lemps(FR)**

Mandataire: **Ramey, Daniel et al**
**Cabinet Ores 6 Avenue de Messine**
**F-75008 Paris(FR)**

Appareil de prise d'informations colorimétriques in situ, en particulier sur une dent en bouche.

Appareil de prise d'informations colorimétriques in situ, comprenant un boîtier (12) dans lequel sont logés une source d'éclairage (18), un système optique (24) de transmission du faisceau lumineux vers l'objet à éclairer, un détecteur (22) de niveau de flux lumineux, un système optique de reprise (34), un spectromètre (40), des photodétecteurs (50), et des circuits électroniques (52) de traitement de signaux, qui sont logés dans la partie arrière (14) du boîtier et reliés à un microprocesseur (56).

L'invention permet notamment la détermination de la couleur des dents d'une personne.

EP 0 367 647 A1

# APPAREIL DE PRISE D'INFORMATIONS COLORIMETRIQUES IN SITU, EN PARTICULIER SUR UNE DENT EN BOUCHE.

L'invention concerne un appareil de prise d'informations colorimétriques in situ, en particulier sur une dent en bouche pour la détermination de la couleur à donner a une prothèse dentaire voisine de la dent.

L'appareil selon l'invention a également d'autres applications, chaque fois qu'il est nécessaire ou utile de déterminer avec précision la couleur d'un objet, notamment pour définir la couleur à donner à un autre objet, identique au premier objet cité ou de même type que celui-ci.

Dans le domaine dentaire, on a proposé d'utiliser des spectrocolorimètres permettant de déterminer, dans la bouche d'un patient, la couleur des dents adjacentes à l'emplacement d'une prothèse, pour donner à cette prothèse une couleur qui réalisera le meilleur accord possible sur le plan esthétique avec les dents adjacentes.

Un tel spectrocolorimètre doit nécessairement être associé à un instrument de prise d'informations colorimétriques sur la dent visée. Il doit donc être aisément manipulable, de préférence à l'aide d'une seule main, et il doit surtout permettre des mesures fidèles et reproductibles de la couleur de la dent. En fait, l'éclairage d'une zone déterminée de la dent, la reprise de la lumière réfléchie et diffusée, son analyse au moyen d'un colorimètre et le traitement des résultats de mesure posent des problèmes qui sont loin d'être résolus dans la technique actuelle et qui se traduisent parfois par des résultats peu satisfaisants au niveau du choix de la couleur donnée à la prothèse, notamment en raison du phénomène de métamérisme (deux couleurs différentes peuvent présenter les mêmes composantes chromatiques sous un éclairage donné et être visuellement identiques, bien que cette identité ne soit pas vérifiée pour d'autres éclairages).

L'invention a pour objet un appareil de prise d'informations colorimétriques in situ, qui permette d'éviter les inconvénients de la technique antérieure.

L'invention a également pour objet un appareil de ce type, qui soit extrêmement compact et dans lequel est intégré l'essentiel des moyens nécessaires à la prise d'informations colorimétriques et au traitement de ces informations, tout en restant aisément manipulable et en ayant des dimensions et une configuration telles que son extrémité puisse être appliquée directement sur la dent dont on veut déterminer la couleur.

L'invention propose donc, à cet effet, un appareil de prise d'informations colorimétriques in situ, en particulier sur une dent en bouche, caractérisé en ce qu'il se présente sous forme d'un boîtier compact manipulable au moyen d'une seule main, comprenant une partie arrière dans laquelle sont logés des circuits électroniques de traitement de signaux, et une partie avant dans laquelle sont logés une source d'éclairage, un système optique de transmission du faisceau lumineux émis par la source, un système optique de reprise de la lumière, et un spectromètre monté entre ce système de reprise et un ensemble de photodétecteurs, ces derniers étant reliés aux circuits électroniques de traitement logés dans la partie arrière.

Un tel appareil est très compact et quasi-autonome, puisqu'il suffit de le relier à une source d'énergie électrique pour le rendre opérationnel.

Selon une autre caractéristique de l'invention, les circuits électroniques de traitement comprennent des circuits de pré-amplification, d'échantillonnage et de conversion analogique-numérique des signaux des photodétecteurs.

Avantageusement, ces circuits comprennent également une interface de liaison à un microprocesseur, ce dernier et ses mémoires pouvant également être logés dans la partie arrière de l'appareil.

On peut également prévoir, sur une des faces du boîtier de l'appareil, un écran plat par exemple à plasma ou à cristaux liquides pour la visualisation des résultats de mesures.

Dans un mode de réalisation préféré de l'invention, la source d'éclairage est une lampe à éclairs, en particulier du type arc Xenon, associée à un circuit électronique d'alimentation et séparée des circuits électroniques de traitement des signaux par une paroi de blindage électromagnétique.

Une telle lampe présente l'avantage d'émettre un faisceau lumineux proche de l'illuminant CIE normalisé D65 correspondant à la lumière du jour. De plus, la répartition spectrale de l'éclair lumineux émis par la lampe est sensiblement constante, et la consommation en énergie est faible.

Selon encore une autre caractéristique de l'invention, cette source lumineuse est associée à un circuit de mesure du flux lumineux émis, qui est relié aux circuits de traitement des signaux.

Selon encore une autre caractéristique de l'invention, le spectromètre est du type à réseau par transmission et est associé à un ensemble de filtres optiques d'équilibrage du niveau du signal lumineux sur les photodétecteurs.

La géométrie linéaire de ce type de spectromètre est favorable à la diminution de l'encombrement de l'appareil.

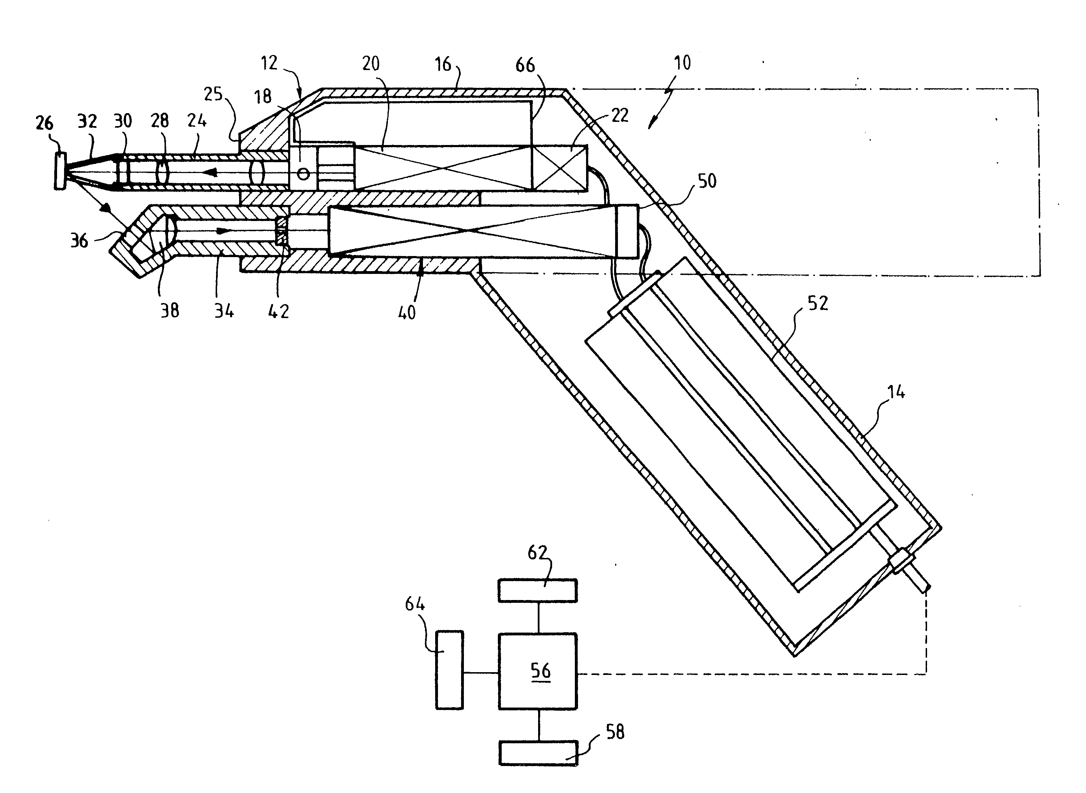

L'invention sera mieux comprise, et d'autres détails, caractéristiques et avantages de celle-ci apparaîtront à la lecture de la description qui suit, faite à titre d'exemple en référence au dessin annexé, dans lequel la figure unique est une vue schématique en coupe d'un appareil selon l'invention.

Cet appareil, désigné généralement par la référence 10, comprend un boîtier 12 constitué essentiellement d'une partie arrière 14 et d'une partie avant 16, faisant entre elles un angle obtus comme représenté en traits pleins, ou bien alignées l'une avec l'autre comme indiqué en traits fantômes.

La partie avant 16 du boîtier contient une source d'éclairage 18, telle qu'une lampe à éclairs du type arc Xenon, associée d'une part à un circuit 20 d'alimentation électrique et à un détecteur 22 de niveau de flux lumineux, et d'autre part à un système optique 24 de transmission du faisceau lumineux vers l'objet visé, par exemple une dent 26 dans la bouche d'un patient.

Le système de transmission 24 comprend un tube cylindrique monté de façon étanche dans un orifice de la paroi 25 d'extrémité avant de la partie 16 de l'appareil, et qui contient des moyens optiques tels qu'une lentille biconvexe 28 transformant le faisceau lumineux émis par la source 18 en un faisceau de rayons sensiblement parallèles, pour éclairer l'objet 26 de façon sensiblement homogène.

L'extrémité avant du tube 24 se termine par un hublot 30 monté de façon étanche, de telle sorte que le système optique de transmission puisse être nettoyé par trempage du tube dans une solution antiseptique.

A l'extrémité avant du système de transmission est également monté un embout 32 d'application sur l'objet 26. Cet embout 32 comprend des ajours importants, de façon à ce que le faisceau lumineux délivré par le système de transmission 24 puisse arriver sur l'objet 26, de façon également à ce que la personne qui tient l'appareil 10 puisse appliquer sans problème l'embout 32 sur la partie visée de l'objet 26, et de façon aussi à ce que la lumière réfléchie et diffusée par l'objet 26 puisse être captée par un système optique de reprise 34 monté de façon étanche dans un orifice de la paroi d'extrémité avant 25 du boîtier.

Le faisceau lumineux délivré par le système 24 et l'embout 32 est sensiblement homogène à rayons parallèles et éclaire une surface relativement importante de la dent, largement supérieure à la surface visée par le système optique de reprise.

Ce système optique de reprise est formé d'un tube comprenant une face d'entrée 36 dont l'axe est orienté à 45° de la direction d'éclairage et derrière laquelle est disposé un prisme 38 à réflexion totale permettant de dévier le faisceau lumineux capté dans une direction parallèle à celle du faisceau lumineux d'éclairage, vers un spectromètre 40 logé dans la partie avant 16 du boîtier 12.

Le spectromètre 40 est du type à réseau par transmission, à géométrie sensiblement linéaire, associé à un système optique de focalisation et à un jeu de filtres ainsi qu'à un ensemble 50 de photodétecteurs, par exemple une barrette de photodiodes.

Les sorties du détecteur 22 de flux lumineux et des photodétecteurs 50 sont reliées à un ensemble de cartes électroniques 52 logées dans la partie arrière 14 du boîtier. Ces cartes sont essentiellement des circuits électroniques formant notamment un circuit de préamplification des signaux des photodétecteurs 50, un circuit d'échantillonnage de ces signaux, et un circuit de conversion analogique-numérique.

Dans l'exemple de réalisation représenté, ces circuits sont reliés, par un cordon 54 traversant le fond de la partie 14, à un microprocesseur 56, extérieur à l'appareil 10, et qui peut être associé à un clavier 58, à une imprimante 60 et à un écran de visualisation 64. De façon classique, le microprocesseur 56 est associé également à des mémoires et à une interface avec les circuits des cartes électroniques 52.

Dans une variante de réalisation, on peut prévoir que le microprocesseur 56, ses mémoires et le circuit d'interface avec les cartes électroniques 52, soient intégrés à l'intérieur de la partie arrière 14, cet ensemble de circuits ayant des dimensions très réduites, et que l'écran de visualisation 64 est un écran plat, par exemple à plasma ou à cristaux liquides, monté sur une face du boîtier 12, par exemple sur la face supérieure de la partie avant 16 ou à l'extrémité de la partie arrière 14.

On voit sur le dessin que le spectromètre 40 se trouve directement en dessous de la source d'éclairage 18 et de son circuit d'alimentation 20. Pour éviter toute perturbation du fonctionnement des détecteurs 22 et 50 et des circuits électroniques portés par les cartes 52, on prévoit une paroi de blindage électro-magnétique 66 autour de la source 18 et de son alimentation 20.

Cet appareil fonctionne de la façon suivante : les circuits et composants divers étant alimentés en énergie électrique, il suffit d'appliquer l'embout 32 contre l'objet visé, par exemple une dent 26, de façon à ce que le faisceau lumineux émis par la source d'éclairage 18 soit sensiblement perpendiculaire à la surface visée de l'objet. Un bouton de commande placé par exemple sur le boîtier 12 permet de déclencher un éclair de durée très courte et de puissance instantanée importante, avec une répartition spectrale correspondant sensiblement à celle de la lumière du jour. La génération d'une puissance lumineuse instantanée importante et la synchronisation de l'émission de l'éclair et de la lecture des photodétecteurs 50 permettent d'éliminer totalement l'influence de la lumière ambiante sur la mesure.

L'éclair lumineux produit par la lampe est transformé en un faisceau de rayons parallèles dans la lentille biconvexe 28 et arrive perpendiculairement sur la surface de l'objet 26. Simultanément, le niveau de flux lumineux émis est mesurée par le détecteur 22.

La lumière réfléchie et diffusée par l'objet 26 est captée, à 45° de la direction d'éclairage, par le prisme 38 à réflexion totale et envoyée vers l'orifice d'entrée du spectromètre 40, de façon à y former une image de la zone visée de l'objet 26. Les filtres optiques intégrés dans le spectromètre permettent d'équilibrer les niveaux de signal reçus par les divers détecteurs, et donc d'homogénéiser la courbe de réponse du système de mesure. Ces filtres permettent également de limiter la lumière parasite sur les détecteurs 50, liée aux différents ordres de diffraction du réseau supérieurs à 1.

Les détecteurs 50, qui sont par exemple des photodiodes au silicium, peuvent être en nombre limité. Dans un mode particulier de réalisation de l'invention, la barrette de photodétecteurs 50 comprend 7 photodétecteurs, permettant d'échantillonner la réflectance spectrale diffuse de l'objet 26 sur sept intervalles de longueur d'onde.

Les signaux des photodétecteurs 50 sont traités par les circuits des cartes électroniques 52 (pré-amplification, échantillonnage, numérisation) et transmis au microprocesseur 56, par exemple pour la détermination et l'enregistrement en mémoire de la réflectance spectrale diffuse de l'objet 26, le calcul des composantes trichromatiques, et le calcul des coordonnées chromatiques dans un espace donné, tel par exemple que (L*, a*, b*).

La mesure du niveau de flux émis par la source d'éclairage à chaque mesure permet, par un simple rapport de niveaux de flux, de rendre les mesures indépendantes du niveau de flux émis. On peut donc utiliser dans l'appareil selon l'invention une source d'éclairage 18 telle qu'une lampe à éclairs, à arc Xenon, qui présente l'inconvénient d'une variation du niveau de flux lumineux lors de l'émission d'éclairs successifs, mais l'avantage de conserver une répartition spectrale sensiblement constante pour les différents éclairs. De plus, une telle lampe a des dimensions réduites, une faible consommation électrique et fournit une puissance lumineuse instantanée importante.

De façon générale, l'invention présente l'avantage de regrouper tous les composants d'une installation industrielle de spectrocolorimétrie en un ensemble léger et compact, manipulable au moyen d'une seule main, et qui permet des mesures in situ sur des objets relativement peu accessibles tels que les dents de la bouche d'un patient.

En outre, une seule prise d'informations sur une dent permet de déterminer la réflectance spectrale de la dent, et non pas uniquement sa couleur apparente pour un éclairage donné.

L'appareil selon l'invention a également l'avantage de respecter les normes relatives aux appareils utilisés en milieu médical (notamment nettoyage et stérilisation simples et rapides, et faible consommation d'énergie électrique). Il peut fonctionner sur accumulateurs ou être relié à un réseau électrique d'alimentation.

## Revendications

1) Appareil de prise d'informations colorimétriques in situ, en particulier sur une dent en bouche caractérisé en ce qu'il se présente sous forme d'un boîtier compact (12) manipulable au moyen d'une seule main, comprenant une partie arrière (14) dans laquelle sont logés des circuits électroniques (52) de traitement de signaux, et une partie avant (16) dans laquelle sont logés une source (18) d'éclairage, un système optique (24) de transmission du faisceau lumineux émis par la source, un système optique (34) de reprise de la lumière, et un spectromètre (40) monté entre ce système de reprise et un ensemble de photodétecteurs (50), ces derniers étant reliés aux circuits électroniques (52) de traitement logés dans la partie arrière (14).

2) Appareil selon la revendication 1, caractérisé en ce que les circuits électroniques de traitement comprennent des circuits de pré-amplification, d'échantillonnage et de conversion analogique-numérique des signaux des photodétecteurs (50).

3) Appareil selon la revendication 1 ou 2, caractérisé en ce que les circuits (52) logés dans la partie arrière (14) comprennent également une interface de liaison à un microprocesseur (56).

4) Appareil selon la revendication 3, caractérisé en ce que les microprocesseurs et ses mémoires sont également logés dans la partie arrière (14).

5) Appareil selon l'une des revendications 1 à 4, caractérisé en ce qu'il comprend, sur une de ses faces, un écran plat, par exemple à plasma ou à cristaux liquides, pour la visualisation des résultats de mesures.

6) Appareil selon l'une des revendications 1 à 5, caractérisé en ce que la source d'éclairage (18) est une lampe à éclairs, par exemple du type arc Xenon, associée à un circuit électronique (20) d'alimentation et séparée des circuits électroniques (52) de traitement des signaux par une paroi (66) de blindage électromagnétique.

7) Appareil selon l'une des revendications précédentes, caractérisé en ce que la source lumineuse (18) est associée à un circuit (22) de mesure du flux lumineux émis, qui est relié aux circuits (52) de traitement de signaux.

8) Appareil selon l'une des revendications précédentes, caractérisé en ce que le système optique de transmission (24) est monté dans un tube cylindrique étanche et se termine par un embout (32) d'application sur l'objet dont il faut déterminer la couleur.

9) Appareil selon la revendication 8, caractérisé en ce que le système optique de transmission (24) et l'embout (32) délivrent un faisceau lumineux sensiblement homogène à rayons parallèles éclairant une surface d'objet, en particulier de dent, largement supérieure à celle visée par le système optique de reprise (34).

10) Appareil selon l'une des revendications précédentes, caractérisé en ce que le système optique de reprise (34) comprend des moyens (38) de déviation du faisceau lumineux et des moyens de formation d'une image d'un objet sur l'orifice d'entrée du spectromètre (40).

11) Appareil selon l'une des revendications précédentes, caractérisé en ce que le spectromètre (40) est du type à réseau par transmission et est associé à un ensemble de filtres optiques (46) d'équilibrage du niveau du signal lumineux sur les photodétecteurs (50) et de suppression des ordres de diffraction supérieurs à 1.

# DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-4 484 819 (R. ULRICH) <br> * Colonnes 1-2 * <br> --- | 1,2,5 | G 01 J 3/50 <br> A 61 C 13/08 |
| X | US-A-4 624 572 (F. VAN DEN BOSCH) <br> * Colonnes 1,3-5 * <br> --- | 1,6,11 | |
| A | US-A-3 421 821 (P. ALESSI) <br> * Colonnes 2-3 * <br> --- | 1,8 | |
| A | US-A-4 758 085 (M. LEQUIME et al.) <br> * Colonne 3,4,6 * <br> --- | 1,2 | |
| A | US-A-3 802 783 (J. SIMMONDS et al.) <br> * Colonnes 3-4 * <br> --- | 1,6 | |
| A | FR-A-1 535 476 (I.N.R.A.) <br> * Pages 2-3 * <br> ----- | 1,7 | |

**DOMAINES TECHNIQUES RECHERCHES (Int. Cl.5)**

G 01 J 3/50
G 01 J 3/46
G 01 N 21/47
A 61 C 13/08

**Le présent rapport a été établi pour toutes les revendications**

| Lieu de la recherche | Date d'achèvement de la recherche | Examinateur |
|---|---|---|
| LA HAYE | 25-01-1990 | BOEHM CH.E.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)